# EUROPEAN PATENT SPECIFICATION

(11) **EP 2 845 898 B1**
(45) Date of publication and mention of the grant of the patent: **05.07.2017**
(21) Application number: 13784860.2
(22) Date of filing: 25.04.2013
(51) Int. Cl.: C12N 5/0797, C12N 11/04, A61K 35/12

(54) **METHOD FOR CULTURING NEURAL CREST STEM CELLS, AND USE THEREOF**
VERFAHREN ZUR KULTIVIERUNG VON NEURALLEISTENSTAMMZELLEN UND VERWENDUNG DAVON
PROCÉDÉ POUR CULTIVER DES CELLULES SOUCHES DE CRÊTE NEURALE, ET UTILISATION DE CELLES-CI

(30) Priority: 04.05.2012 KR 20120047702
(43) Date of publication of application: 11.03.2015
(73) Proprietor: Inje University Industry-Academic Cooperation Foundation, Gimhae-si, Gyeongsangnam-do 621-749 (KR)
(72) Inventor: YANG, Young Il, Busan 612-741 (KR); LEE, Seung Jin, Seoul 150-937 (KR); KIM, Jong Tae, Busan 617-763 (KR); CHEONG, Soon Ho, Busan 608-890 (KR)
(74) Representative: Roos, Peter
(86) International application number: PCT/KR2013/003547
(87) International publication number: WO 2013/165120

(56) References cited:
- CA-A1- 2 529 003
- KR-B1- 100 811 537
- US-A- 5 824 489
- US-A1- 2003 099 623
- US-A1- 2003 099 623
- US-A1- 2006 281 177
- US-B2- 8 030 072
- HONG-YUN LI ET AL: "Isolation and Characterization of Neural Crest Progenitors from Adult Dorsal Root Ganglia", STEM CELLS., vol. 25, no. 8, 24 May 2007 (2007-05-24), pages 2053-2065, XP055223855, US ISSN: 1066-5099, DOI: 10.1634/stemcells.2007-0080
- EL SEADY R ET AL: "Uncomplicated differentiation of stem cells into bipolar neurons and myelinating glia", BIOCHEMICAL AND BIOPHYSICAL RESEARCH COMMUNICATIONS, ACADEMIC PRESS INC. ORLANDO, FL, US, vol. 376, no. 2, 14 November 2008 (2008-11-14), pages 358-362, XP025495739, ISSN: 0006-291X, DOI: 10.1016/J.BBRC.2008.08.166 [retrieved on 2008-09-17]
- O P FLINT: "A MICROMASS CULTURE METHOD FOR RAT EMBRYONIC NEURAL CELLS", J. CELL SCI, vol. 61, 1 January 1983 (1983-01-01), pages 247-262, XP055224420,
- Edward M. Davis: "Translocation of neural crest cells within a hydrated collagen lattice", Journal of Embryology and Experimental Morphology, 1 February 1980 (1980-02-01), 2013, pages 17-31, XP055176172, ENGLAND Retrieved from the Internet: URL:http://dev.biologists.org/content/55/1 /17.abstract [retrieved on 2015-10-28]
- YOUNG-IL YANG ET AL: "Ex vivo organ culture of adipose tissue for in situ mobilization of adipose-derived stem cells and defining the stem cell niche", JOURNAL OF CELLULAR PHYSIOLOGY, vol. 224, no. 3, 1 September 2010 (2010-09-01), pages 807-816, XP055223516, US ISSN: 0021-9541, DOI: 10.1002/jcp.22188
- KIM JONG-TAE ET AL: "A fibrin-supported myocardial organ culture for isolation of cardiac stem cells via the recapitulation of cardiac homeostasis", BIOMATERIALS, vol. 48, 10 February 2015 (2015-02-10), pages 66-83, XP029141954, ISSN: 0142-9612, DOI: 10.1016/J.BIOMATERIALS.2015.01.041
- DAVIS, EM.: 'Translocation of neural crest cells within a hydrated collagen lattice' J. EMBRYOL. EXP. MORPH. vol. 55, 1980, pages 17 - 31, XP055176172

## Description

### [Technical Field]

The present invention relates to a method for culturing adult peripheral nerve-derived neural crest stem cells and a use thereof.

### [Background Technology]

Neural stem cells were known to exist only in fetus in the initial period of the developmental stage. However, their presence in mature mammals even after the birth was recently identified. For the last 100 years, it was an orthodox that in post-birth mammals, neurons are not newly generated. Recently, however, the presence of neural stem cells in hippocampus or subventricular zone of the adult central nervous system was reported, and differentiation of the adult neural stem cell into an injured neuron or oligodendrocyte to replace lost or injured neuron or oligodendrocyte was also reported.

However, regarding adults, neural stem cells are isolated and cultured only after a tissue source is collected from a central nervous system, such as the brain or spinal cord. However, regarding adults, hippocampus or subventricular zone, which are known as a place where neural stem cells exist, is located deeply inside of the human brain, and thus, collecting of tissues may cause fatal adverse effects that may cause death. As a result, the difficulty in use of neural stem cells as a self neural stem cell agent for the treatment of Parkinson's Disease, stroke, amyotrophic spinal cord lateral sclerosis, spinal cord injury, motor nerve injury, or trauma-derived central neurologic or peripheral neurologic disorders is getting attention.

To overcome the tissue supply issue of neural stem cells, research into how to use non-nerve tissue-originated hematopoietic stem cells, mesenchymal stem cells, or umbilical blood cells as a cell therapeutic agent for neurologic disorder has been performed. However, although there were experimental results showing that the non-nerve tissue-originated stem cell is differentiated into some neurons, the differentiation potential was extremely limited and accordingly, the use of the non-nerve tissue-originated stem cell as an ideal agent for the treatment of neurologic disorders was limited.

During the migratory stage of the developmental stage, neural crest stem cells migrate from a dorsal part of neural fold, and neural crest stem cells migrate to a wide range of tissues and organs in the body. After the migration, neural crest stem cells are differentiated into neurons of peripheral nervous system, glial cells, melanocytes of the skin, endocrine cells, and various mesenchymal cells, and distributed in adult non-nerve tissues and organs.

Recently, it was disclosed that even after birth, neural crest stem cells exist in peripheral nerves, dorsal root ganglia, gut, hair follicle, dermal papillae, cornea, peripheral nerves, and dental pulp. In particular, only in hair follicle, dermal papillae, and dental pulp, neural crest stem cells of adult human beings were able to be separated. However, since dermal papillae or hair follicle has various epidermal cell-derived appendages, cells that form neurosphere from corresponding tissues are highly likely to be mixed with foreign cells derived from skin appendages. Accordingly, such cells are not suitable for use as a cell therapeutic agent.

Also, although conventional research results did prove that neural crest stem cells exist even in adult human beings, conventional technology requires 3-month long cell culturing to separate and culture neural crest stem cells, and due to the long-term culturing, various dysplasia, such as transformation or dedifferentiation, may occur. Also, according to conventional technology, a particular marker is used to isolate neural crest stem cells. However, up until now, there are no markers that specifically represent neural crest stem cells.

Conventionally, the following three methods are used to isolate neural crest stem cells from adult human beings. According to a first method, adult dermal papillae, hair follicle, or dental pulp is treated with histolytic enzyme, such as collagenase, dispase, or trypsin, to isolate a single cell group dissociated from a solid tissue: a tissue dissociation step. The obtained single cells are amplified by using a monolayer culture method, neurosphere is formed, and then, only a cell that forms neurosphere is isolated therefrom and then, cultured. According to the second method, a solid tissue is subjected to a tissue dissociation step in the same manner as explained in the first method, and then, single dissociated cells are isolated, and by using p75 marker or CD140a marker, only cells expressing corresponding markers are purified by using an immunological method, and then, are amplified by using a monolayer culture method. According to the third method, dermal papillae, hair follicle, or dental pulp fragment is seeded onto a culture plate by explant culture, and then, secondarily, cells that migrate from a tissue fragment to the culture plate and proliferate in the culture plate are purified by immunological method, or neurosphere forming cells are purified.

However, these methods of isolating and culturing neural crest stem cells have several problems. First, the isolation of single dissociated cells from neural crest-originated tissue necessarily requires tissue dissociation. The tissue dissociation has a large variation in the population[yield] of obtained single cells according to a histolytic enzyme used, potency, a reaction time, a reaction temperature, or the state of a tissue used. As a result, it is difficult to standardize tissue dissociation, and also, the tissue dissociation has low isolation yield and low efficiency. Second, single dissociated cells obtained by tissue dissociation include various heterogeneous cells that constitute a neural crest tissue. Single dissociated cells isolated from the tissue further include, in addition to neural crest stem cells, a vascular endothelial cell, vascular smooth muscle cells, adipocytes, blood cells, and fibroblast. To selectively isolate neural crest stem cells from the heterogeneous single dissociated cells, an immunological purification method using a marker that specifically expresses only in a neural crest stem cell is needed. However, such a marker that specifically expresses only in a neural crest stem cell has not been disclosed. Third, neurosphere is not a feature that occurs only in a neural crest stem cell. According to conventional research results, the structure of neurosphere is also observed in adipose-derived stem cells, mesenchymal stem cells, endothelial progenitor cells, and hematopoietic stem cells. Accordingly, this method is not appropriate for the purification of neural crest stem cells.

### [Detailed Description of the Invention]

### [Technical Field]

The present invention provides a method of culturing a peripheral nerve-derived neural crest stem cell, wherein the method includes culturing a peripheral nerve fragment inserted into the hydrogel.

The peripheral nerve-derived neural crest stem cells cultured according to the culturing method of the present invention have at least one immunological profile selected from the group consisting of (i) an immunological characteristic that is positive to at least one of neural crest stem cell markers selected from the group consisting of nestin, p75, sox-10, and CD56; (ii) an immunological characteristic that is positive to at least one of mesenchymal stem cell markers selected from the group consisting of CD29, CD44, CD73, CD90, and CD105; (iii) an immunological characteristic that is positive to at least one of pericyte markers selected from the group consisting of CD140b, CD146, and SMA (α-smooth muscle actin); (iv) an immunological characteristic that is negative to at least one of hematopoietic cell markers selected from the group consisting of CD34 and CD45; (v) an immunological characteristic that is negative to at least one of vascular endothelial cell markers selected from the group consisting of CD31 and CD34; and (vi) an immunological characteristic that is negative to at least one of nervous system cell markers selected from the group consisting of NF (neurofilament), S-100, GFAP (glial fibrillary acid protein), and MBP (myelin basic protein).

### [Technical Solutions]

The present invention provides a method of culturing peripheral nerve-derived neural crest stem cells, wherein the method includes: culturing peripheral nerve fragments after inserting the peripheral nerve fragments into the hydrogel; and breaking down only the hydrogel to collect neural crest stems cell that have migrated into the hydrogel from the peripheral nerve fragments and proliferated in the hydrogel.

The peripheral nerve may be the peripheral nerve obtained from an adult body.

The peripheral nerve-derived neural crest stem cells may have at least one the immunological profile characteristic selected from the group consisting of (i) an immunological characteristic that is positive to at least one of neural crest stem cell markers selected from the group consisting of nestin, p75, sox-10, and CD56; (ii) an immunological characteristic that is positive to at least one of mesenchymal stem cell markers selected from the group consisting of CD29, CD44, CD73, CD90, and CD105; (iii) an immunological characteristic that is positive to at least one of pericyte markers selected from the group consisting of CD140b, CD146, and SMA (α-smooth muscle actin); (iv) an immunological characteristic that is negative to at least one of hematopoietic cell markers selected from the group consisting of CD34 and CD45; (v) an immunological characteristic that is negative to at least one of vascular endothelial cell markers selected from the group consisting of CD31 and CD34; and (vi) an immunological characteristic that is negative to at least one of nervous system cell markers selected from the group consisting of NF (neurofilament), S-100, GFAP (glial fibrillary acid protein), and MBP (myelin basic protein).

The hydrogel may be collagen hydrogel, and the hydrogel may include 0.5 to 5.0 mg/ml of collagen.

The breaking down of the collagen hydrogel may be performed by treating the collagen hydrogel with collagenase.

An example of the method of culturing a peripheral nerve-derived neural crest stem cells includes 1) a first step of inserting the peripheral nerve fragment into the collagen hydrogel; 2) a second step of adding a growth culture medium thereto to perform a three-dimensional organ culture in a shaker having a speed of 5 to 30 rpm ; 3) a third step of treating with collagenase to break down only the collagen hydrogel to collect the peripheral nerve fragment and neural crest stem cells which have migrated into the hydrogel from the peripheral nerve fragment and proliferated in the collagen hydrogel; and 4) a fourth step of amplifying the collected neural crest stem cell by monolayer culture.

The peripheral nerve fragment collected in the third step is inserted into the hydrogel of the first step and is cultured.

The peripheral nerve-derived neural crest stem cells cultured according to the culturing method of the present invention have at least one immunological profile selected from the group consisting of (i) the immunological profile that is positive to at least one of neural crest stem cell markers selected from the group consisting of nestin, p75, sox-10, and CD56; (ii) an immunological characteristic that is positive to at least one of mesenchymal stem cell markers selected from the group consisting of CD29, CD44, CD73, CD90, and CD105; (iii) an immunological characteristic that is positive to at least one of pericyte markers selected from the group consisting of CD140b, CD146, and SMA (α-smooth muscle actin); (iv) an immunological characteristic that is negative to at least one of hematopoietic cell markers selected from the group consisting of CD34 and CD45; (v) an immunological characteristic that is negative to at least one of vascular endothelial cell markers selected from the group consisting of CD31 and CD34; and (vi) an immunological characteristic that is negative to at least one nervous system cell markers selected from the group consisting of NF (neurofilament), S-100, GFAP (glial fibrillary acid protein), and MBP (myelin basic protein).

The neural crest stem cells may have a differentiation potential into a neurons, including oligodendrocytes, astrocytes, Schwann cells, osteoblasts, chondrocytes, adipocytes, or skeletal muscle cells.

A method of differentiating a peripheral nerve-derived neural crest stem cell into nervous system cellscan include culturing the neural crest stem cells by micromass.

A cell therapeutic agent can include, as an active ingredient, the neural crest stem cells or cells differentiated therefrom.

The cell therapeutic agent is a therapeutic agent for at least one of neuronal cells selected from oligodendrocytes, astrocytes, Schwann cells, osteoblasts, and adipocytes.

The cell therapeutic agent may further include at least one of active ingredients selected from the group consisting of anti-inflammatory drugs, stem cell recruitment factors, and an angiogenic factors.

The neural crest stem cells may be mixed with hydrogel.

A pharmaceutical composition for the prevention or treatment of neurologic disorder caninclude, as active ingredients, neural crest stem cells or cells differentiated therefrom.

The neurologic disorder may be degenerative neurologic disorders, demyelination neurologic disorder, amyotrophic lateral sclerosis, traumatic spinal cord disorder, or peripheral neural disorders.

### [Advantageous Effects]

Adult peripheral nerve-derived neural crest stem cells according to the present invention have excellent in vitro proliferation capacity, and have a multipotency of being able to be differentiated into cells that constitute the peripheral nervous system and the central nervous system. Accordingly, neural crest stem cells are suitable for use in the production of new drugs, such as cell therapeutic agent, tissue-engineering therapeutic agent, and tools for cytological, molecular biological fundamental research and the new drug development research, associated with the recruitment, migration, proliferation, and differentiation into nervous system cells from neural crest stem cells.

### [Brief Description of Drawings]

FIG. 1 shows migration and proliferation of neural crest stem cells into the collagen hydrogels prepared with different concentrations of collagen from the peripheral nerve.
FIG. 2 shows the degree, assessed by morphometric method, of migration and proliferation of neural crest stem cells in the collagen hydrogels prepared with different concentrations of collagen. (*, p < 0.01 compared to 5.0 and 10.0 mg/ml group; #, p < 0.05 compared to 2.6, 5.0 and 10.0 mg/ml group).
FIG. 3 shows low magnification phase-contrast microscopic images (x 40 magnification) of a three-dimensional organ culture of collagen hydrogel-supporting peripheral nerve fragment.
FIG. 4 shows high magnification phase-contrast microscopic images of a three-dimensional organ culture of collagen hydrogel-supporting peripheral nerve fragment.
FIG. 5 shows migration and proliferation of neural crest stem cells under collagen hydrogel-supporting three-dimensional peripheral nerve fragment, according to culture conditions. (Dynamic, a dynamic culture condition at 15 rpm; Static, a static culture condition; *, p < 0.01 compared to the static group).
FIG. 6 shows a phase-contrast microscopic image of neural crest stem cells in a monolayer culture condition after being separated from collagen hydrogel.
FIG. 7 shows colony formation of neural crest stem cells in monolayer culture condition after being separated from collagen hydrogel.
FIG. 8 shows colony formation frequency of neural crest stem cells in monolayer culture condition after being separated from collagen hydrogel.
FIG. 9 shows population doubling time (PDT) of a neural crest stem cells in monolayer culture condition after being separated from collagen hydrogel.
FIG. 10 shows the immunophenotypic characteristics of a neural crest stem cell in monolayer culture condition after being separated from collagen hydrogel, analyzed by flow cytometry.
FIG. 11 shows the immunophenotypic characteristics of a neural crest stem cells in monolayer culture condition after being separated from collagen hydrogel, obtained by analyzing by immunofluorescence staining.
FIG. 12 shows the mRNA expression characteristics of neural crest stem cells in monolayer culture condition after being separated from collagen hydrogel.
FIG. 13 shows the differentiation potential of neural crest stem cells in monolayer culture condition after being separated from collagen hydrogel into osteoblasts (A), adipocytes (B), astrocytes (D), neurons (E).
FIG. 14 shows the differentiation potential of neural crest stem cells in a monolayer culture condition after being separated from collagen hydrogel into Schwann cells.
FIG. 15 shows immunofluorescence staining images of neural crest stem cells induced to differentiate into Schwann cells.
FIG. 16 shows the mRNA expression characteristics of neural crest stem cells induced to differentiate into Schwann cells.
FIG. 17 shows staining images showing the peripheral nerve regeneration capacity of neural crest stem cells in a sciatic nerve injury animal model.
FIG. 18 shows amplitude results of a nerve conduction test showing nerve function recovery of neural crest stem cells in a sciatic nerve injury animal model.
FIG. 19 shows results of a nerve conduction test showing nerve function recovery by neural crest stem cells in a sciatic nerve injury animal model.
FIG. 20 shows immunofluorescence staining images showing differentiation of neural crest stem cells into Schwann cells in a sciatic nerve injury animal model.

### [Best Mode]

The present invention provides a method of culturing neural crest stem cells derived from a peripheral nerve.

Hereinafter, the present invention will be described in detail.

Conventionally, hematopoietic stem cells or mesenchymal stem cells are used to treat intractable peripheral neural disorders. However, it is difficult to amplify and culture hematopoietic stem cells in vitro, and the isolation and purification of the effective dosage of hematopoietic stem cells require 10 L of bone marrow. To obtain mesenchymal stem cells, conventionally, in the case of a solid organ, tissue dissociation and secondary purification of mesenchymal stem cells are required. The obtained mesenchymal stem cells may exist in one of a million of nucleated cells in bone marrow or adipocyte tissue, and less than 1 % of marrow or adipocyte tissue-derived mesenchymal stem cells may form a colony. In addition, a time taken for division of one cell into two cells is 60 hours or more. That is, it is difficult to amplify cells in vitro to obtain the effective cell therapeutic agent dosage. Furthermore, it is known that the hematopoiesis-originated stem cells is not directly differentiated into neurons. Thus, these stem cells are not suitable for use as cell therapeutic agents for neurologic disorders. The inventors of the present invention made efforts to solve these problems, and found that according to the method according to present invention, it was confirmed that a neural crest stem cells exists even in the adult peripheral nerve, and neural crest stem cells having excellent in vitro expansion capacity and differentiation potential into nervous system cells were able to be obtained in high yield from peripheral nerve fragment even without tissue dissociation of peripheral nerve and secondary purification of neural crest stem cells , thereby completing the present invention.

The present invention provides a method of culturing peripheral nerve-derived neural crest stem cells, wherein the method includes: culturing peripheral nerve fragment after embedding the peripheral nerve fragment into the hydrogel; and breaking down only the hydrogel to collect neural crest stem cells that has migrated into the hydrogel from the peripheral nerve fragment and proliferated in the hydrogel.

An example of a method of culturing peripheral nerve-derived neural crest stem cells includes 1) a first step of embedding the peripheral nerve fragment into the collagen hydrogel; 2) a second step of adding growth culture medium thereto to perform a three-dimensional organ culture in a shaker having a speed of 5 to 30 rpm ; 3) a third step of treating with collagenase to break down only the collagen hydrogel to collect the peripheral nerve fragment and neural crest stem cells which have migrated into the hydrogel from the peripheral nerve fragment and proliferated in the collagen hydrogel; and 4) a fourth step of amplifying the collected neural crest stem cells by monolayer culture.

The peripheral nerve is a representative neural crest-originated organ, and is highly accessible due to its wide distribution in the human body, and small tissue thereof is able to be harvested from an adult patient via minimum invasive surgery to provide a stable tissue supply for the isolation and culture of a cell therapeutic agent. Also, the peripheral nerve, unlike other organs, mostly consists of neural crest-originated cells, and thus, is less likely contaminated with heterogeneous cells. Accordingly, the peripheral nerve is advantageous in the isolation and culture for the use as cell therapeutic agent for neurologic disorders.

An origin of the peripheral nerve is not particularly limited, and for example, may be a mammal including human beings.

In addition, a harvesting age of the peripheral nerve is not particularly limited, and for example, the peripheral nerve may be the peripheral nerve of the adult body. According to conventional technology, neural crest stem cells are isolated from the peripheral nerve of fetus to new-born babies, not from the peripheral nerve of the adult body. However, a method of culturing according to the present invention enables isolation and culturing of neural crest stem cells derived from the peripheral nerve of the adult human body.

A fragment of the peripheral nerve may be a fragment obtained by slicing the peripheral nerve. For example, the soft tissue and epineurium surrounding the peripheral nerve is removed by using a scissors and then, the resultant peripheral nerve is sliced to obtain a fragment having a size of 1 to 3 mm³.

The hydrogel is a three-dimensional network structure in which the hydrophilic polymer is crosslinked via a conjugated or non-conjugated bond, and has phase-transition ability, and in a solution state, the hydrogel is uniformly mixable with the peripheral nerve fragment, and after the mixing, the hydrogel is phase-transitioned into the solid gel to provide the three-dimensional physical support to the peripheral nerve fragment, and at the same time, to provide the three-dimensional cell attachment substrate in which neural crest stem cells in the peripheral nerve fragment attache, migrate, and proliferate.

In the case in which the hydrogel provides the three-dimensional cell attachment substrate, the supply of the three-dimensional substrate that allows the neural crest stem cells to continuously migrate into and proliferate in the hydrogel is confirmed by providing an integrin-β1 binding receptor that is needed to the migration and proliferation of neural crest stem cells from the peripheral nerve fragment.

Integrin-β1 in the cells bind the extracellular matrix, after then, FAK is phosphorylated to activate the cellular signal transduction pathway, resulting in initiation of cell division. In detail, when the peripheral nerve fragments attach to the wider area of the extracellular matrix, the cellular signal transduction pathway has higher activity and thus, cell division increases. In this regard, the hydrogel provides the wider three-dimensional cell attachment substrate than the two-dimensional cell attachment substrate provided by monolayer culture so that sufficient space for the migration and proliferation of neural crest stem cells is provided, leading to high cell division, suppression of intercellular contact inhibition, and a long-term culture.

The hydrogel is not limited to a particular kind of hydrogel, and may include the hydrogel produced from at least one of selected from the group consisting of natural polymer, synthetic polymer, and a copolymer produced by mixing various polymers. For example, the hydrogel may include at least one of selected from the group consisting of collagen, gelatin, fibrin, chondroitin, hyaluronic acid, alginic acid, Matrigel™, chitosan, and peptide. For example, the hydrogel may include collagen hydrogel.

The hdyrogel composed of synthetic polymer or copolymer does not break down for a long period of time, thereby having excellent physical properties. However, such hdyrogel has poor biological properties, such as decreasing of migration and proliferation. On the other hand, the hydrogel composed of natural polymer, such as fibrin, has biocompatibility, such as increasing of cell migration and proliferation. However, the hydrogel composed of natural polymer has poor physical properties, such as faster breaking-down than the hydrogel composed of synthetic polymer or copolymer.

However, the collagen hydrogel according to the present invention is hydrogel composed of natural polymer showing biocompatibility, such as increasing of cell migration and proliferation, and at the same time, has a resistance to an action of matrix metalloproteinase or fibrinolytic enzyme released from the nerve fragment. Accordingly, during culturing, the collagen hydrogel does not break down and stably provides the cell attachment substrate. That is, although other natural polymer hydrogels, such as fibrin, are easily dissolved due to the action of a fibrinolytic enzyme to fail to hold the supporting function of the hydrogel, the collagen hydrogel has a resistance to the fibrinolytic enzyme and due to the resistance, the collagen hydrogel may be the most appropriate for culturing the peripheral nerve-derived neural crest stem cells.

When the peripheral nerve fragment according to the present invention is cultured in collagen hydrogel, collagen concentration is not particularly limited, and may be, for example, in a range of 0.5 to 5.0 mg/ml, and 1.0 to 3.5 mg/ml.

For example, the collagen hydrogel may be prepared using 0.5 to 5.0 mg/ml of collagen and 5 to 50 mM sodium bicarbonate.

When the collagen concentration is greater than the upper limit of the range described above, the collagen hydrogel may have a compact structure and lower porosity in its structure. In this case, however, migration and proliferation of cells are seriously decreased. On the other hand, when the collagen concentration is smaller than the lower limit of this rang described above, the formed hydrogel may break down due to matrix metalloproteinase that is secreted from cells and peripheral nerve fragment during culturing. Thus, the hydrogel may not further act as the three-dimensional cell attachment substrate needed for the migration and proliferation of cells.

The growth culture medium may indicate themedium that induces recruitment, migration, and proliferation of neural crest stem cells in vitro, and may include the conventional medium that is used in culturing mammalian cells. According to an embodiment of the present invention, Dulbecco's modified Eagle medium (DMEM), RPMI, Hams F-10, Hams F-12, α-Minimal Essential Medium (α-MEM), Glasgow's Minimal Essential Medium, and Iscove's Modified Dulbecco's Medium, which are commercially prepared cell culture mediums, may be used.

According to the present invention, the growth culture medium may further include a proliferation factor that induces the recruitment, migration, and proliferation of neural crest stem cells in vitro. The proliferation factor may include a serum, that is, a serum of an animal including human beings, a basic fibroblastic growth factor (bFGF), vascular endothelial growth factor (VEGF), insulin, an epidermal growth factor (EGF), a leukemia inhibitory factor (LIF), an insulin-like growth factor (IGF), a platelet-derived growth factor (PDGF), a stem cell factor (SCF), or the like. The growth culture medium may include antibiotics, such as penicillin, streptomycin, or gentamicin.

In an embodiment of the present invention, the growth culture medium may include a DMEM:HamsF12 (1:1) culture, a fetal bovine serum, EGF, bFGF, IGF, and gentamicin. In detail, the growth culture medium may include a 70 to 95 (vol/vol) % DMEM:Hams F12 (1:1) culture, a 5 to 15 (vol/vol)% fetal bovine serum, a 5 to 50 ng/ml EGF, a 0.5 to 10 ng/ml bFGF, a 5 to 50 ng/ml IGF, and a 5 to 50 mg/ml gentamicin.

As a conventional three-dimensional culture of organ fragment, there are two methods: organ fragment is exposed in air to promote an exchange of oxygen and nutrients; and organ fragment is dynamically cultured on an orbital shaker. In the case of the exposing of the organ fragment in air, however, the supply of nutrients is limited. Accordingly, the exposing method is not appropriate for a long-term culture. In the case of the dynamic culture, due to the swirling of a culture medium, the organ fragment exposed to the culture medium may have shearing injury.

However, according to the present invention, an organ is cultured while being in the hydrogel. Since the peripheral nerve fragment is present in the hydrogel, without shearing injury, neural crest stem cells may receive sufficient amounts of oxygen and nutrients by shaking to promote proliferation thereof.

A shaking speed is not particularly limited. For example, the shaking speed may be in a range of 5 to 30 rpm. When the shaking speed is outside the lower limit of the range, oxygen and nutrients may not be sufficiently supplied, and when the shaking speed is outside the upper limit of the range, the peripheral nerve fragment may be shearing injured, and the culture of neural crest stem cells in the hydrogel may be instable.

To selectively break down only the hydrogel, a polymer-specific breakdown enzyme that may selectively break down a polymer that constitutes the hydrogel may be added into the culture medium. The polymer-specific breakdown enzyme is not particularly limited, and may include at least one of selected from the group consisting of collagenase, gelatinase, urokinase, streptokinase, plasmin, and hyaluronidase.

According to an embodiment of the present invention, when the peripheral nerve fragment according to the present invention is three-dimensional organ cultured by using collagen hydrogel, gelatin hydrogel, or Matrigel hydrogel, collagenase or gelatinase is added into the culture medium to selectively break down the hydrogel; when the hydrogel consisting of fibrin is used, at least one selected from the group consisting of urokinase, streptokinase, and plasmin is added into the culture medium to selectively break down the hydrogel consisting of fibrin; and when hyaluronic acid hydrogel is used, hyaluronidase may be used to selectively break down the hydrogel.

According to the present invention, the adding of the hydrogel constituting polymer-specific breakdown enzyme into the culture medium does not lead to any structural destruction or cytotoxicity of neural crest stem cells and a peripheral nerve fragment, and 95% or more of collected neural crest stem cells survive.

In an embodiment, for the selective breaking-down of collagen or gelatin hydrogel, 0.1 to 5 mg of collagenase may be added into the culture medium per 1 ml of collagen or gelatin hydrogel. A collagenase concentration may be appropriately determined as long as only collagen hydrogel is selectively breakdown without breaking down of the structure of neural crest stem cells or peripheral nerve fragment.

In an embodiment, the breaking-down effects of collagenase on hydrogel may be enhanced by carrying out the reaction at a temperature of 30 to 37°C for 30 minutes to 3 hours.

After the selective breaking down of hydrogel by the treatment with the breakdown enzyme, neural crest stem cells that migrate into and proliferate in the hydrogel and peripheral nerve fragment that is not structurally injured may be collected.

The neural crest stem cells collected from the hydrogel may be expanded. The expanding method is not particularly limited, and may be a monolayer culture method. According to an embodiment of the present invention, neural crest stem cells collected from the hydrogel is subjected to the subculture: neural crest stem cells are seeded on a culture plate, and then when the neural crest stem cells reach 60 to 90% confluence of the surface area of the culture plate, the neural crest stem cells are treated with trypsin-EDTA to detach them from the culture plate, and then, the neural crest stem cells are seeded on a new culture plate. Due to the subculture, the neural crest stem cells may be expandable until a desired population of cells are obtained.

In some embodiments, peripheral nerve fragment that is collected by selectively breaking down the hydrogel is embedded back into the hydrogel to repeatedly perform a three-dimensional organ culture to continuously isolate and culture neural crest stem cells. Since only the hydrogel is selectively broken down after the culturing and collecting, the peripheral nerve fragment may retain its original structure.

As described above, in a method of culturing neural crest stem cells using hydrogel according to the present invention, after a small amount of peripheral nerve is collected once from a patient with neurologic disorders, the peripheral nerve is able to be continuously cultured. Accordingly, without re-collecting of peripheral nerve, neural crest stem cells may be isolated in an amount that is needed to isolate peripheral or central nervous system disorders.

According to the present invention, a three-dimensional organ culture period of the peripheral nerve fragment is not particularly limited. For example, the three-dimensional organ culture period may be in a range of 1 to 28 days, for example, 7 to 20 days. In the organ culture of the hydrogel-supporting three-dimensional peripheral nerve fragment, 12 hours after the culture begins, neural crest stem cells are able to be induced to migrate from the peripheral nerve fragment into hydrogel and to proliferate in the hydrogel.

Neural crest stem cells obtained by using a method of culturing peripheral nerve may have at least one immunological profile selected from the group consisting of (i) an immunological characteristic that is positive to at least one of neural crest stem cell markers selected from the group consisting of nestin, p75, sox-10, and CD56; (ii) an immunological characteristic that is positive to at least one of mesenchymal stem cell markers selected from the group consisting of CD29, CD44, CD73, CD90, and CD105; (iii) an immunological characteristic that is positive to at least one of pericyte markers selected from the group consisting of CD140b, CD146, and SMA (α-smooth muscle actin); (iv) an immunological characteristic that is negative to at least one of hematopoietic cell markers selected from the group consisting of CD34 and CD45; (v) an immunological characteristic that is negative to at least one of vascular endothelial cell markers selected from the group consisting of CD31 and CD34; and (vi) an immunological characteristic that is negative to at least one of nervous system cell markers selected from the group consisting of NF (neurofilament), S-100, GFAP (glial fibrillary acid protein), and MBP (myelin basic protein).

According to conventional methods, cells that constitute peripheral nerve are isolated as a single cell by the treatment with a tissue dissociation enzyme, and then, an immunologic method is necessarily used to isolate neural crest stem cells from a heterogeneous cell group. However, it is difficult to standardize tissue dissociation, and single cells obtained may have a large variation in the population according to the type, potency, a reaction time, and a reaction temperature of the tissue dissociation enzyme used, and the state of a tissue used. In addition, during tissue dissociation, cell injury is inevitable, leading to low cell isolation yield. However, a method of culturing neural crest stem cells according to the present invention is simple, and a method does not include tissue dissociation and thus, cell injury does not occur. Also, 95% or more of collected cells survive, and when 100 mg of peripheral nerve fragment cultured for 14 days, 1.7 x 10⁶ cells are obtained. Accordingly, unlike conventional methods, a culturing method according to the present invention enables the isolation and culturing of neural crest stem cells in large quantities.

The peripheral nerve-derived neural crest stem cells cultured according to the culturing method of the present invention have at least one immunological profile selected from the group consisting of (i) an immunological characteristic that is positive to at least one of neural crest stem cell markers selected from the group consisting of nestin, p75, sox-10, and CD56; (ii) an immunological characteristic that is positive to at least one of mesenchymal stem cell markers selected from the group consisting of CD29, CD44, CD73, CD90, and CD105; (iii) an immunological characteristic that is positive to at least one of pericyte markers selected from the group consisting of CD140b, CD146, and SMA (α-smooth muscle actin); (iv) an immunological characteristic that is negative to at least one of hematopoietic cell markers selected from the group consisting of CD34 and CD45; (v) an immunological characteristic that is negative to at least one of vascular endothelial cell markers selected from the group consisting of CD31 and CD34; and (vi) an immunological characteristic that is negative to at least one of nervous system cell markers selected from the group consisting of NF (neurofilament), S-100, GFAP (glial fibrillary acid protein), and MBP (myelin basic protein).

In a culturing method of neural crest stem cells, neural crest stem cells that migrate into and proliferate in hydrogel may express at least one of neural crest stem cell markers selected from the group consisting of nestin, p75, sox-10, and CD56, and the marker may be positively expressed in 95% or more of fetus and new-born babies.

Neural crest stem cells may express at least one of markers selected from the group consisting of CD29, CD44, CD73, CD90, and CD105, which are markers of mesenchymal stem cell, and at least one marker selected from the group consisting of CD140b, CD146, and SMA, which are pericyte markers. However, a contamination with somatic cells, such as vascular endothelial cells, hematopoietic cells, or smooth muscle cells, that constitute a peripheral nerve may occur at less than 1%, and s-100, MBP, GFAP, and NF, which are mature neuron markers, may not be expressed, Lin, CD34, and CD45, which are hematopoietic cell markers, may be negative, and CD31 and CD34, which are vascular endothelial cell markers, may not be expressed.

The neural crest stem cells may have a differentiation potential into mesenchymal cells. In an embodiment, the neural crest stem cells may have a differentiation potential into neurons, oligodendrocytes, astrocytes, Schwann cells, osteoblasts, chondrocytes, adipocytes, or skeletal muscle cells.

The neural crest stem cells may be directly differentiated into neurons. However, a direct differentiation of hematopoietic stem cells and mesenchymal stem cells into peripheral neurons has not been proved.

Also, conventionally, neural crest stem cells are isolated from the peripheral nerve of a fetus and new-born, but not from the peripheral nerve of the adult human being. However, according to the present invention, peripheral nerve-derived neural crest stem cells are isolated from the human adult body and cultured, and the isolated and cultured neural crest stem cells show a differentiation potential into neurons, oligodendrocytes, astrocytes, and Schwann cells.

From among neural crest stem cells that are cultured and isolated by using a culturing method according to the present invention, 35 to 47% constitute a colony, show about 100 times of cell division, and have a doubling time, which is a time required for a single cell to divide into two cells, of 30 to 60 hours. That is, these neural crest stem cells have excellent in vitro proliferation capacity. In contrary, from among bone marrow or adipose tissue-derived mesenchymal stem cells obtained by using conventional culturing methods, only less than 1% forms a colony, and the doubling time is 60 hours or more. Accordingly, according to the present invention, due to excellent in vitro proliferation capacity, neural crest stem cells may be obtained for a relatively short period of time in an amount that is effective for the treatment of organ disorders.

The neural crest stem cells may be mesenchymal stem cells, and may be cells that has differentiation characteristics equivalent to those of a central nervous system-originated neural stem cells. The neural crest stem cells are not particularly limited, and in an embodiment, may be differentiated into neurons, oligodendrocytes, astrocytes, Schwann cells, osteoblasts, chondrocytes, adipocytes or skeletal muscle cells.

A cell into which the neural crest stem cell is voluntarily differentiated is not particularly limited. For example, the neural crest stem cells may be differentiated into neurons, oligodendrocytes, astrocytes, or Schwann cells which constitute peripheral and central nervous systems.

A method of differentiating peripheral nerve tissue-derived neural crest stem cells into nervous system cells can include culturing the neural crest stem cells by micromass.

The nervous system cells differentiated from neural crest stem cells are not limited, and may include at least one selected from the group consisting of neurons, oligodendrocytes, astrocytes, and Schwann cells, and for example, Schwann cells.

Conventionally, differentiation of neural crest stem cells into peripheral neuron has been carried out by differentiation into Schwann cells by using BMP4, Nrg1, or Delta-Fc in monolayer culture condition [Development 2004;131:5599]. However, this conventional method is limited in differentiation inducing efficacy into peripheral neurons, and is not effective to confirm a differentiation potential of a particular stem cells into peripheral neurons. All cells and tissues in vivo are present in a three-dimensional structure, and their functions are maintained due to an intercellular interaction and a cell-extracellular matrix interaction. However, the 2-dimensional monolayer culture condition differs from in vivo condition, and does not maintain the intercellular interaction and the cell-extracellular matrix interaction. Accordingly, the monolayer culture condition has a limitation in inducing differentiation into the peripheral neuron.

However, the micromass culturing according to the present invention may induce cells to align in a three-dimensional fine structure via the intercellular interaction and the cell-extracellular matrix interaction. In an embodiment, 100,000 neural crest stem cells are suspended in 10 ml of cell culture medium, and then, seeded onto a culture plate, and then, the neural crest stems cell may be induced to be aligned in the three-dimensional structure. An inducing method may be as follows: cells are induced to align a three-dimensional structure and then for one day to 4 weeks, for example, for one day to four weeks, for example, for three days to two weeks, thereby inducing differentiation into peripheral neurons. A micromass culturing method may support, via the three-dimensional structure, the seeded intercellular interaction and the cell- extracellular matrix interaction.

In addition, a micromass culturing method may be used as a system for evaluating differentiation potential of somatic cells, embryonic stem cells, or reprogrammed pluripotent stem cells, which are able to be differentiated into Schwann cells, into Schwann cells, and also used as a system for evaluating differentiation inducing capacity of a material or factor that may induce differentiation into Schwann cells.

Schwann cells produced by differentiation due to the micromass culturing may have an immunological characteristic that is positive to at least one of markers selected from myelin basic protein (MBP), and S100, glial fibrillary acid protein (GFAP).

A cell therapeutic agent can include, as an active ingredient, the neural crest stem cells or cells differentiated therefrom.

For use the cell therapeutic agent, neural crest stems cell may be directly used without any particular differentiation process, or may be differentiated into target cells for the cell therapy before use.

Cells used in the cell therapeutic agent are not particularly limited, and in an embodiment, may include at least one of cells selected from the group consisting of neurons, oligodendrocytes, astrocytes, Schwann cells, osteoblasts, and adipocytes.

A method of using neural crest stem cells or cells differentiated therefrom as the cell therapeutic agent is not particularly limited. Any conventional method may be used herein. For example, the neural crest stem cells may be supplied while being carried on a biodegradable scaffold or a vehicle.

The biodegradable scaffold or vehicle may be any one of various materials that do not induce a substantial harmful reaction in a host, are biologically decomposable, are naturally removable from a biological system, and/or are chemically mixable with a biological system.

The biodegradable scaffold or vehicle are not particularly limited, and may be, for example, hydrogel.

The neural crest stem cells may be mixed with hydrogel and then the resultant structure is used as the cell therapeutic agent. An available hydrogel is not particularly limited, and may be hydrogel consisting of a natural polymer, for example, a collagen hydrogel. In an embodiment, when collagen hydrogel is used, collagen concentration may be adjusted to control a breaking down speed in vivo. The hydrogel may prevent loss of neural crest stem cells into blood when neural crest stem cells are embedded, and also, cell injury due to inflammatory cell and enzyme at injured sites may be reduced.

The cell therapeutic agent may additionally include at least one selected from the group consisting of anti-inflammatory drugs, stem cell recruitment factors, and angiogenic factors.

The anti-inflammatory drug may be used to protect neural crest stem cells in the hydrogel from excessive inflammatory reaction, and due to the inclusion of the stem cell recruitment factors or the angiogenic factors, recruitment of stem cell and vascular growth in peripheral nerve are induced to enhance cell regeneration effects.

The anti-inflammatory drug may not particularly limited, and in an embodiment, may include at least one selected from the group consisting of COX inhibitor, ACE inhibitor, salicylate, and dexamethasone.

The the stem cell recruitment factor may not be particularly limited, and in an embodiment, may include at least one selected from the group consisting of IGF, bFGF, PDGF, and EGF.

The angiogenic factor may not be particularly limited, and in an embodiment, may include at least one selected from EGF, PDGF, VEGF, ECGF, and angiogenin.

A pharmaceutical composition for the prevention or treatment of neurologic disorder caninclude, as an active ingredient, the neural crest stem cells or cells differentiated therefrom.

The neurologic disorders may include central nervous and peripheral nervous system disorders. For example, the neurologic disorders may be degenerative neurologic disorder, demyelination neurologic disorder, an amyotrophic lateral sclerosis, traumatic spinal cord disorder, or peripheral neural disorder.

Conventionally, hematopoietic stem cells or mesenchymal stem cells are conventionally used as the therapeutic agent for an intractable neurologic disorder. For use as the cell therapeutic agent that is applicable to a patient of a neurologic disorder, for example, degenerative neurologic disorder, demyelination neurologic disorder, amyotrophic lateral sclerosis, traumatic spinal cord disorder, or peripheral neural disorder, 10 million to 100 million cells are needed. In the case of hematopoietic stem cell, in vitro amplification is difficult, and 10 L of marrow is required to isolate and purify an effective capacity of hematopoietic stem cell. The mesenchymal stem cell is known such that one mesenchymal stem cell exist per a million of nucleated cells in bone marrow or adipose tissue, and bone marrow or adipose -derived mesenchymal stem cell forms a colony at less than 1%, and time taken for a single cell to divide into two cells is 60 hours or more. Accordingly, the difficulty in amplification in vitro to obtain an effective capacity of a cell therapeutic agent is regarded as a limitation in use as a cell therapeutic agent. Also, it has not been proved whether hematopoietic stem cells and mesenchymal stem cells are directly differentiated into peripheral neuron.

According to the culturing method according to the present invention, at least two million neural crest stem cells are able to be cultured within 14 days from 100 mg of peripheral nerve, and due to a 2-week monolayer culture, at least 10 million neural crest stem cells are amplified. Also, 30% of the cultured neural crest stem cells form a colony, and show at least 100 times of cell division ability, and a time required for a single cell to divide into two cells is in a range of 30 to 60 hours. As described above, the culturing method according to the present invention provides excellent in vitro proliferation ability. Accordingly, within a short period of time, an effective amount appropriate for use as a cell therapeutic agent may be obtained.

The neural crest stem cells included in the pharmaceutical composition are not particularly limited, and may be mixed with hydrogel before use.

The peripheral nerve-derived neural crest stem cells according to the present invention have in vitro expandable capacity, and spontaneous differentiation potential into nervous system cells. Accordingly, the neural crest stem cells are effectively used as a composition for the prevention or treatment of central nervous and peripheral nervous system disorders.

As described above, it is confirmed that neural crest stem cells injected into injured peripheral nerve is directly differentiated into Schwann cells, that is, a nervous system tissue.

The pharmaceutical composition includes, as an active ingredient, the neural crest stem cells or cells differentiated therefrom. The pharmaceutical composition may further include a carrier, an excipient, and a diluent which are conventionally used in the preparation of the pharmaceutical composition. The pharmaceutical composition may be prepared by using a conventional method that is available in the art (see Remington's Pharmaceutical Science, the newest edition; Mack Publishing Company, Easton PA).

The administration method of the pharmaceutical composition is not limited. In an embodiment, the pharmaceutical composition may be non-orally administrated. For example, the pharmaceutical composition may be intraneurally administrated. However, the present invention is not limited thereto.

The pharmaceutical composition may further include at least one selected from the group consisting of anti-inflammatory drugs, stem cell recruitment factors, and angiogenic factors.

### [Embodiments]

Hereinafter, embodiments of the present invention will be described in detail. However, these embodiments are only an example of the present invention, and do not limit the scope of the present invention.

### <Examples>

### Example 1 Migration and proliferation of neural crest stem cells from peripheral nerve according to collagen concentration

Collagen (Bioland, Osong, The Republic of Korea) extracted from Bovine skin was dissolved in 0.1 % (wt./vol.) acetic acid to prepare 1.0, 2.0, 5.0, 10.0, and 20.0 mg/ml collagen solutions. Collagen hydrogel reconstitution buffer was prepared using 50 mM NaHCO₃, 40 mM HEPES, and 0.01 N NaOH. Peripheral nerves donated by a brain-dead patient were used in the present experiments. By using a scissors, peripheral nerve was cut to remove a surrounding soft tissue and epineurium therefrom, and then, sliced to obtain fragments thereof each having a size of 1 to 3 mm³. The fragments were washed three times with DMEM solution. Next, the peripheral nerve fragments were added such that 10 fragments were included in 0.5 ml of hydrogel reconstitution buffer. Thereafter, the five different concentrations of collagen solution and the peripheral nerve fragment-containing reconstitution buffer were mixed at a ratio of 1:1 (capacity), and 1 ml of the mixed solution was transferred onto a 6-multiwell tissue culture plate, and then subjected to a crosslinking reaction in a humidified chamber at a temperature of 37°C for 2 hours. When collagen hydrogel was completely formed, 2 ml of cell culture medium was added thereto, and then, a skeletal muscle fragment was organ-cultured in a three-dimensional hydrogel for 7 days. When the culture was fully grown, the cells were fixed using 3% formalin solution, and then, photographed by using a phase-contrast microscope. Then, a migration distance of neural crest stem cells from the peripheral nerve fragment to collagen hydrogel was measured by using Image J(NIH, Bethesda, MA).

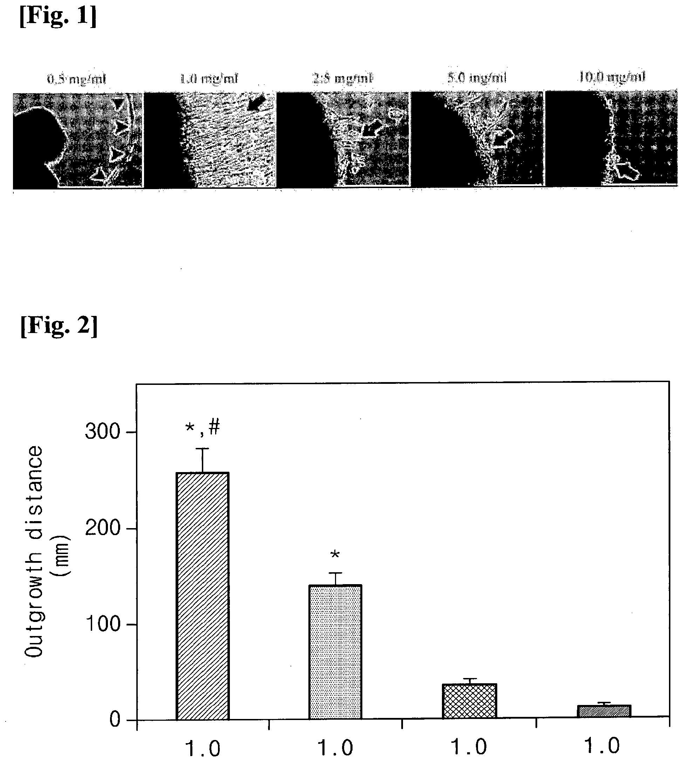

Referring to FIGS. 1 and 2, when the collagen concentration is 0.5 mg/ml or less, collagen hydrogel breaks down due to matrix metalloproteinase secreted by peripheral nerve and thus, a substrate needed for the adhesion and migration of three-dimensional cell collapses (see an arrowhead shown in FIG. 1). When the collagen concentration increases, the migration and proliferation of neural crest stem cells in the hydrogel are substantially reduced (see FIGS.1 and 2). The migration and proliferation of neural crest stem cells from peripheral nerve fragment to the hydrogel produced using 1.0 mg/ml collagen was observed from the first culture day. However, the migration of neural crest stem cell into hydrogel prepared using 5 mg/ml concentration or more was observed from the second culture day. Even in morphometric assay, when collagen concentration used to prepare hydrogel increased, the migration and proliferation of neural crest stem cell were substantially decreased (p < 0.01)(FIG. 2).

### Example 2 Collagen hydrogel-supporting three-dimensional organ culture of peripheral nerve fragment

Peripheral nerves donated by a brain-dead patient were used in the same manner as in Example 1. Peripheral nerve fragments, each having a size of 1 to 3 mm³, from which epineurium and perineural soft tissue were removed, were mixed with 7.5 ml of hydrogel reconstitution buffer. Then, the result was mixed with the same amount of 2 mg/ml collagen solution, and then 15 ml of the mixed solution was transferred onto a 150-mm culture plate, and in a humidified chamber, subjected to a crosslinking reaction at a temperature of 37°C for 2 hours. When collagen hydrogel was completely formed, 20 ml of cell culture medium was added thereto and then organ-cultured. The cell culture medium consisted of 90% (vol/vol) DMEM, 10% fetal bovine serum(FBS; Invitrogen), 20 ng/ml EGF, 5 ng/ml bFGF, 10 ng/ml IGF, and 10 mg/ml gentamicin (Invitrogen). After the addition of the cell culture medium, culturing was carried out for 14 days while the culture plate was rotated on an orbital shaker at a speed of 15 to 30 rpm speed. On every two days, the cell culture medium was replaced with fresh cell culture medium. A neural crest stem cell which had migrated into and proliferated in hydrogel and the peripheral nerve fragment in hydrogel were treated with collagenase to selectively break down collagen hydrogel. Collected neural crest stem cells were expanded by using a conventional two-dimensional monolayer culture method, and the collected peripheral nerve fragment was embedded back into the collagen hydrogel, and then, the hydrogel-supporting three-dimensional organ culture was performed thereon.

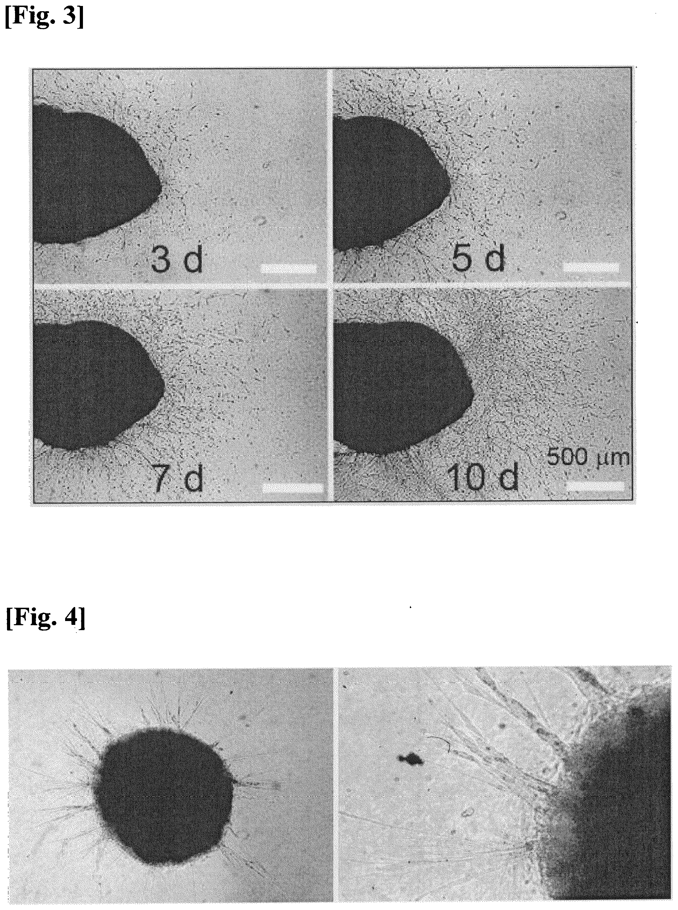

As shown in FIGS. 3 and 4, from the first day of organ culture, the migration and proliferation of neural crest stem cells from peripheral nerve fragments into hydrogel were observed, and up to the 14^{th} day of the culture, the migration and proliferation of neural crest stem cells into collagen hydrogel were continuously increased. Cells that migrated into and proliferated in hydrogel had a spindle shape that is similar to that of a conventional fibroblasts (see FIG.4).

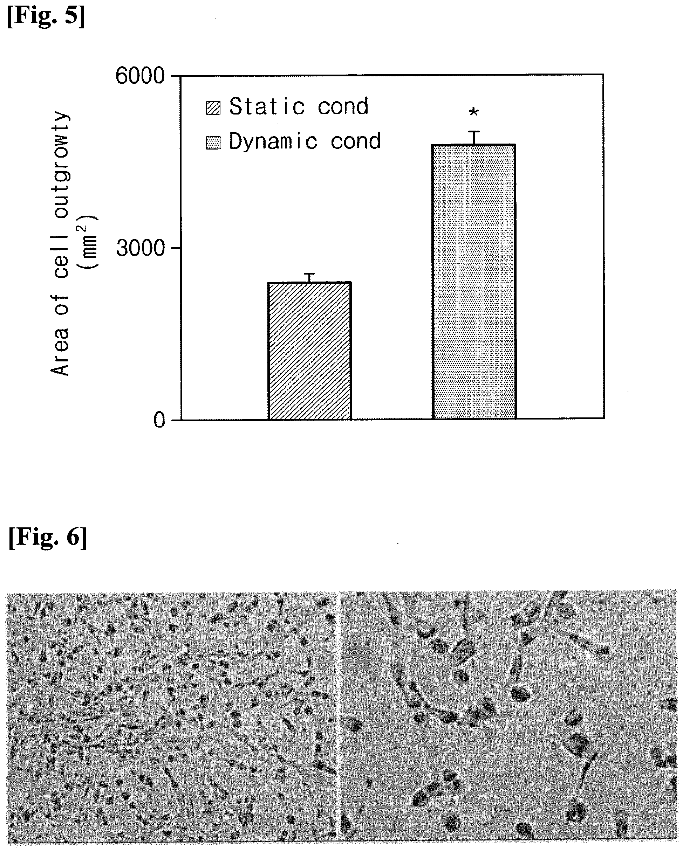

Referring to FIG. 5, it was confirmed that in a dynamic condition, the collagen hydrogel-supporting three-dimensional organ culture promotes supply of oxygen and nutrients. Accordingly, in comparison of organ culture in a static condition, the migration and proliferation of neural crest stem cells from peripheral nerve fragment to hydrogel in the dynamic condition significantly increases by 30% or more (p < 0.01) (see FIG.5).

### Example 3 Collection and amplification of neural crest stem cells that migrated and proliferated in collagen hydrogel

Fragments of peripheral nerve were cultured for 2 weeks in the same manner as explained in Example 2. A culture plate was washed three times with phosphate-buffered saline (PBS) for 10 minutes at room temperature, and then, washed once with 1 % calf serum-containing DMEM. DMEM including 1 % calf serum and collagenase type-I(Worthington Biochemical Co., Lakewood, NJ) was loaded into the culture plate in such a way that the amount of DMEM was 20 ml in every 10 ml of collagen hydrogel. The culture plate was placed on an orbital shaker and rotated at a temperature of 37°C and at a speed of 15 rpm for 30 minutes. When collagen hydrogel was loosened and broken down, neural crest stem cells and peripheral nerve fragments were transferred from the culture plate to 50 ml conical tubes by using a transfer pipette. Thereafter, centrifuging was performed at 200 x g for 10 minutes and then, a supernatant was removed. 10 ml of cell culture medium was added thereto, and then, dispersed by using a transfer pipette. A cell strainer having a pore size of 100 mm (BD Bioscience, Seoul, and The Republic of Korea) was used to separate neural crest stem cells and peripheral nerve fragments. The population of collected cells was analyzed by using PicoGreen dsDNA Quantitation Kit, and adjusted in consideration of the weight of the peripheral nerve that was organ-cultured. The neural crest stem cells were centrifuged at 200 x g for 10 minutes, and then a supernatant was removed therefrom and a cell pellet was dispersed into the cell culture medium. The neural crest stem cells were seeded at a density of 10,000 cells per cm², and then, amplified by using a conventional monolayer culture method.

30 minutes after treatment with collagenase, collagen fiber was broken down, and in neural crest stem cells, cytoplasm shrunk to be round and was dispersed individually. These cells were able to be easily collected by centrifuging because hydrogel was dispersed in a solution state after breaking down. When the collected cells were seeded onto a cell culture plate, the neural crest stem cells were attached in the form of a single layer to the surface of the culture plate within 30 minutes and proliferated (FIG.6). The population of neural crest stem cells obtained from 100 mg of peripheral nerve fragment after collagen hydrogel-supporting three-dimensional organ culture for 14 days was as high as 1.7 x 10⁶.

### Example 4 Proliferation characteristics of neural crest stem cell collected from collagen hydrogel

To measure a forming ability of colony forming unit-fibroblast (CFU-F), the cells collected from hydrogel were seeded on a 60-mm culture dish at a density of 5 cells/cm², and then, a proliferation culture medium was added thereto and the cells were cultured for 10 days. Thereafter, cells in the culture dish were fixed with 2% buffered formalin and then subjected to staining with 10% crystal violet (LabChem Inc., Pittsburgh, PA) for 5 minutes. An image of a stained culture dish was obtained by using a digital CCD camera, and the population of CFU-F was counted by using an image analysis program (Image J). In addition, to measure population doubling time (PDT) of neural crest stem cells collected from the hydrogel, 2 x 10³ cells were seeded onto a 48-multiwell culture plate, and then cultured. On the first and fifth culture day, the cells were lysed in CelLytic™ solution (Sigma), and DNA content in the lysed sample was measured by using PicoGreen dsDNA Quantitation Kit. Fluorescent microplate reader (Synergy™ HT; Bio-Tek Instruments, Neufahrn, Germany) was used to measure a fluorescent intensity at an emission wavelength of 485 nm and an excitation wavelength of 540 nm. To convert the measured fluorescence intensity into cell population, a standard curve was obtained by using fibroblast, and the standard curve was used to convert the fluorescent intensity in a sample into cell population. PDT was determined as follows: PDT=[(days in exponential phase)/((logN2-logN1)/log2)], wherein N1 is a cell population in an initial period in an exponential growth phase, and N2 is a cell population in a terminal period in the exponential growth phase.

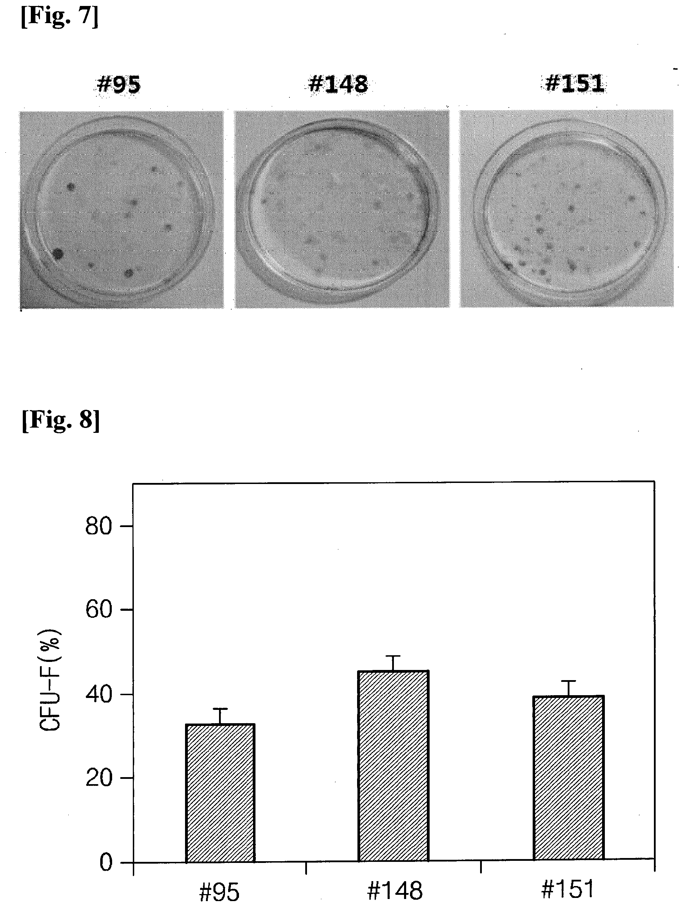

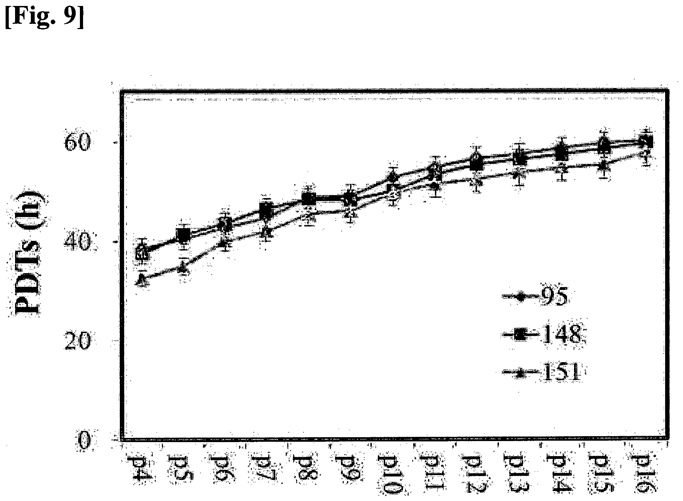

FIGS. 7, 8, and 9 show proliferation characteristics of neural crest stem cells collected from collagen hydrogel in a monolayer culture condition. Neural crest stem cells collected from collagen hydrogel were able to form CFU-F at 35 % to 47 % (see FIGS. 7 and 8). Neural crest stem cells were able to be subcultured 16 times or more, and to carry out cell division 96 times or more (FIG. 9). Also, the population doubling time of neural crest stem cells was within 30 hours to 60 hours. Thus, it was confirmed the cells have excellent dell division capacity in a monolayer culture condition (FIG. 9).

### Example 5 Phenotypic characteristics of neural crest stem cells collected from collagen hydrogel

### 1) Immunophenotypic characteristics of neural crest stem cell assayed by flow cytometry

100,000 cells were reacted with primary antibodies or isotype-matched control antibodies for 30 minutes. Primary antibodies were anti-human antibodies conjugated with phycoerythrin (PE) or fluorescein isothiocyanate (FITC), and for use as anti-human antibodies, CD29, CD31, CD34, CD44, CD45, CD73, CD90, and CD133 were used. All primary antibodies and control antibodies conjugated with a fluorescent material were obtained from BD Bioscience (San Jose, CA). CD105 (R&D Systems), PDGFR-β(CD140b; Abcam, Cambridge, MA), CD56(Abcam), major histocompatibility complex-I and II(MHC-I and II, Abcam), a-smooth muscle actin (DAKO, SMA), or S-100 (Sigma), which were unconjugated antibodies that were not conjugated with a fluorescent marker, were reacted with 100,000 cells for 30 minutes, and then with FITC-conjugated with secondary antibodies for 30 minutes. When the reaction stopped, the cells were washed three times with PBS, and then, fixed in 2% paraformaldehyde. A production rate with respect to each antibody was analyzed by using FACSCalibur (Becton Dickinson, San Jose, CA) flow cytometry, and an expression rate of at least 10,000 cells was analyzed.

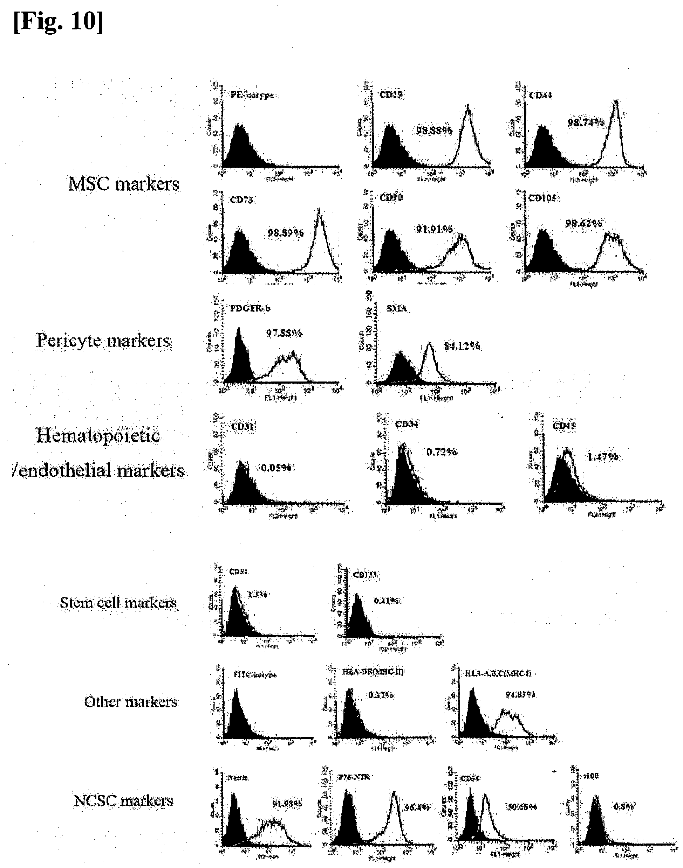

Referring to FIG. 10, neural crest stem cells were positive to nestin, p75, and CD56 which were neural crest stem cell markers. However, CD34 and CD133, which were hematopoietic stem cell markers, were not detected. Also, in the case of neural crest stem cells which were amplified by subculture, 90% thereof were positive to CD29, CD44, CD73, CD90, and CD105 which were mesenchymal stem cell markers, but 100% thereof were negative to CD34 and CD45 which were hematopoietic cell markers and CD31 and CD34 which were vascular endothelial cell markers. 95% or more of the neural crest stem cells were positive to MHC-II marker and negative to MHC-I marker. That is, neural crest stem cells satisfied immunological conditions for heterogeneous transplantation. A pericyte marker, such as CD140b and SMA, was expressed in neural crest stem cells, but the expression frequency slightly varied according to a marker. However, S-100, which is a mature oligodendrocyte or Schwann cell marker, was not expressed.

### 2) Immunophenotypic characteristics of neural crest stem cell assayed by immunofluorescence staining

100,000 neural crest stem cells were attached to a glass slide by using a cytocentrifuge (Cyto-Tek, Sakura, Tokyo, Japan). Expression of nestin, p75, Sox10, CD56 and the like, which are neural crest stem cell markers, was evaluated by immunofluorescence staining. The cells on the slide were reacted with the antibodies, and then, with isotype-matched Alexa Fluor 488-conjugated secondary antibodies. Then, nuclei of the cells on the slide were stained by using DAPI (Invitrogen), and then confirmed by using a confocal microscope. A total of 1000 cells were selected, and an expression rate of corresponding markers in neural crest stem cells was evaluated.

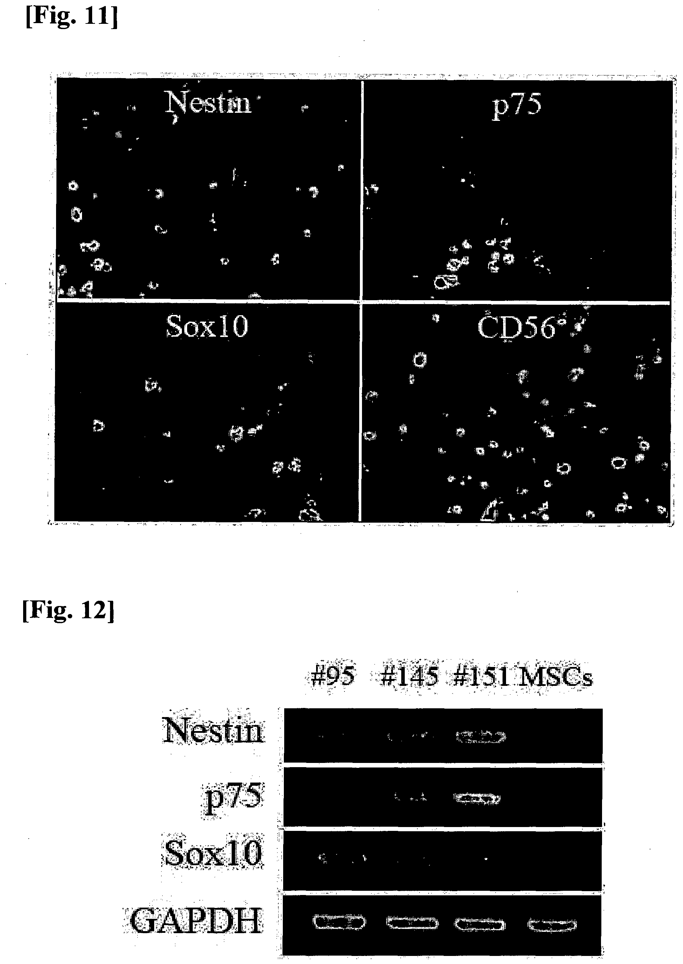

FIG. 11 shows an immunofluorescence staining image of neural crest stem cells that migrated into and proliferated in collagen hydrogel. In the case of cells which were amplified by using the monolayer culture method, 90% or more thereof expressed nestin, p75, Sox10, and CD56 which are neural crest stem cell markers.

### 3) mRNA expression characteristics of neural crest stem cells assayed using reverse transcription polymerase reaction

Total RNA was extracted from 100,000 neural crest stem cell by using TRI Reagent (Invitrogen). cDNA was synthesized by using reverse transcriptase, and then, was subjected to a polymerase reaction using nestin, p75, Sox10 specific primer. Electrophorolysis was performed in 1 % agarose gel, and then, mRNA expression was evaluated.

FIG. 12 shows an electrophorolysis image showing mRNA expression in neural crest stem cells that migrated into and proliferated in collagen hydrogel. Cells that were amplified by a monolayer culture method, expressed nestin, p75, Sox10, and CD56, which are neural crest stem cell mRNA markers, and cells that migrated into and proliferated in hydrogel showed the same mRNA expression characteristics as those of neural crest stem cells.

### Example 6 Multipotency of neural crest stem cells amplified by using a monolayer culture method

### 1) Differentiation potential into osteoblasts

To evaluate differentiation potential of neural crest stem cells into osteoblasts, 20,000 cells were seeded onto a 24-multiwell tissue culture plate, and then, cultured in a a-MEM containing 1 µM dexamethasone, 50 µM ascorbic acid, 10 mM β- glycerol phosphate, and 10% calf serum for 2 weeks to induce differentiation. The differentiation into osteoblasts was confirmed in the following manner: 2 weeks after the induced differentiation, the cells were fixed in 2% formalin at room temperature for 10 minutes, and then, Alizarin Red (Sigma) staining was performed thereon to confirm whether minerals precipitated.

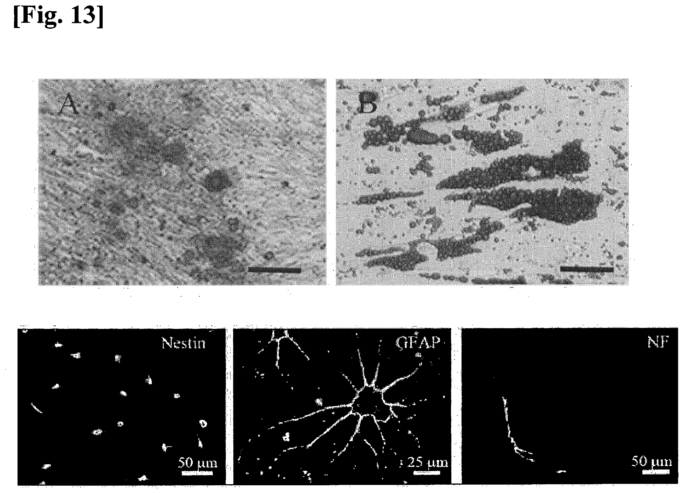

Referring to FIG. 13A, after the induced differentiation of neural crest stem cells, it was observed that mineral precipitated in a wide range of area. Accordingly, it was confirmed that neural crest stem cells have a differentiation potential into osteoblast.

### 2) Differentiation potential into adipocytes

To evaluate differentiation potential of neural crest stem cells into adipocytes, 20,000 cells were seeded onto a 24-multiwell tissue culture plate, and then cultured in a medium including 90% DMEM supplemented with 10% calf serum, 0.5 mM 3-isobutyl-1-methylxanthine (Sigma), 80 µM indomethacin (Sigma), 1 µM dexamethasone (Sigma), and 5 *µ*g/ml insulin (Sigma) for 14 days. The differentiation into adipocyte was confirmed in the following manner: 2 weeks after the induced differentiation, the cells were fixed in 2% formalin at room temperature for 10 minutes, and then, subjected to staining using 0.5% Oil Red O(Sigma) solution, which is an index for intracellular fat deposit, at room temperature for 1 hour to confirm deposits of lipids in cytoplasm.

Referring to FIG. 13B, from the third day after the induced differentiation of neural crest stem cells into adipocytes, the deposit of lipids in cytoplasm was observed, and on the 14th day after the induced differentiation, in at least 90% cells, the deposits of lipids in cytoplasm was confirmed by using Oil Red O staining.

### 3) Differentiation potential into nervous system cells

To induce differentiation into a nervous system cell, 20,000 neural crest stem cells were inserted into a 300 *µ*ℓ fibrin hydrogel consisting of 2.5 mg/ml fibrinogen and 0.5 U/ml thrombin, and then, three-dimensionally cultured. The differentiation into neurons was carried by adding a neuron differentiation medium including 98.5% DMEM, 1% calf serum, 0.5% DMSO, 10 ng/ml BMP-2(R&D systems), 10 ng/ml EGF, and 10 ng/ml bFGF and culturing for 7 days. Differentiation into neuroglia cells was carried out by adding a neuroglia cell differentiation medium including 98.5% DMEM, 1% calf serum, 0.5%DMSO, 10 ng/ml NRG-1 (R&D systems) and culturing for 7 days. The differentiation into neuron and neuroglia cell was confirmed by immunofluorescence staining. To inhibit a non-specific reaction of antibody, the cells were blocked using 0.4% triton-X 100 solution-containing 5% goat serum for 40 minutes, and then, the cells were reacted with neurofilament (abcam, NF), which is a neuron marker, or GFAP(DAKO) antibody, which is a neuroglia cell marker, at a temperature of 37°C for 2 hours. Then, the cells were washed three times with PBS, and then reacted with Dylight 488-conjugated secondary antibodies at room temperature for 50 minutes, and nuclei was stained using a 10 µg/ml DAPI (4',6-diamidino-2-phenylindole, Invitrogen) solution. Thereafter, a ProLongGold antifade reagent (Molecular Probe) was mounted, and expression of the markers was confirmed by using a confocal laser scan microscope.

As shown in FIG. 13, after the induced differentiation into nervous system cells, it was confirmed that thin cytoplasm grew in neural crest stem cells (FIG. 13C), and the neural crest stem cells changed into star-shaped (FIG. 13D), or two-extreme shaped (FIG. 13E) cells. When neural crest stem cells were differentiated, nestin was continuously expressed, and GFAP (FIG. 13D), which is a neuroglia cell marker, or neurofilament (FIG. 13E), which is a neuron marker, was expressed.

### 4) Differentiation potential into Schwann cells by using micromass culture

To induce differentiation into Schwann cell, 100,000 neural crest stem cells were suspended in 10 ml culture, and then, seeded onto a 24-multiwell culture plate. The neural crest stem cells were reacted in a cell incubator for 2 hours, and then, a Schwann cell differentiation medium including 98.5% DMEM, 1% calf serum, 0.5%DMSO, and 10 ng/ml TGF-β2(R&D systems) was added thereto, and then the neural crest stem cells were cultured for 7 days. The differentiation into Schwann cells was confirmed in the following manner: a paraffin block was prepared according to a conventional method, and then, a paraffin fragment was collected therefrom and confirmed by hematoxylin eosin staining and immunofluorescence staining. To inhibit a non-specific reaction of antibodies, paraffin fragment was blocked using 0.4% triton-X 100 solution-containing 5% goat serum for blocking for 40 minutes, and then, reacted with antibodies, such as p75, nestin, S-100, myelin basic protein (MBP), or GFAP, a temperature of 37°C for 2 hours. Then, the cells were washed three times with PBS, and then reacted with Dylight 488-conjugated secondary antibodies at room temperature for 50 minutes, and nuclei was stained using a 10 µg/ml DAPI (4',6-diamidino-2-phenylindole, Invitrogen) solution. Thereafter, a ProLongGold antifade reagent (Molecular Probe) was mounted, and expression of the markers was confirmed by using a confocal laser scan microscope. From micromass which was formed after the induced differentiation into Schwann cell, total RNA was extracted by using a TRI reagent (Invitrogen) according to a conventional method. After cDNA was synthesized using reverse transcriptase, a specific primer was used to carry out a polymerase reaction to evaluate expression of S-100, MBP, Sox-10, GFAP specific mRNA.

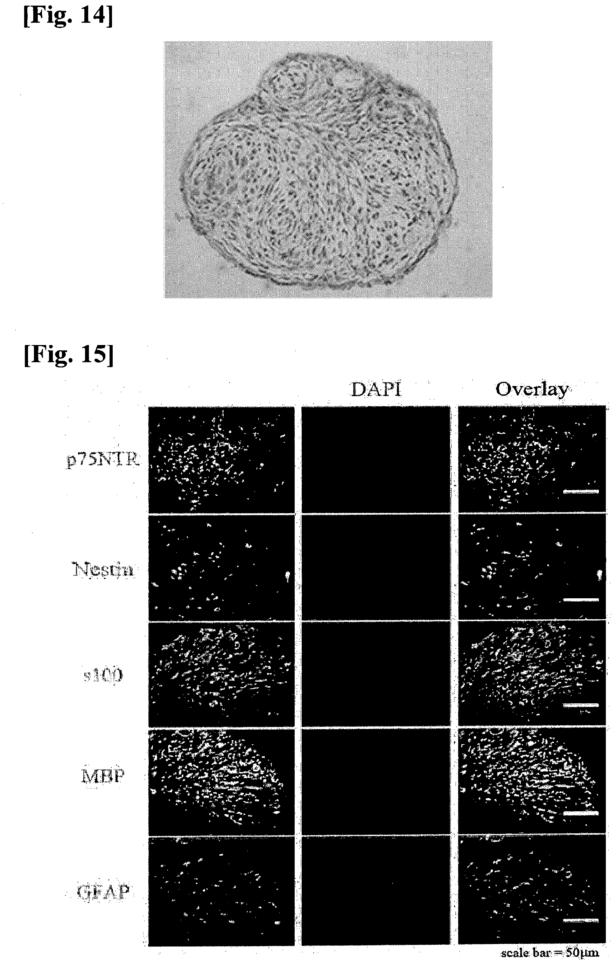

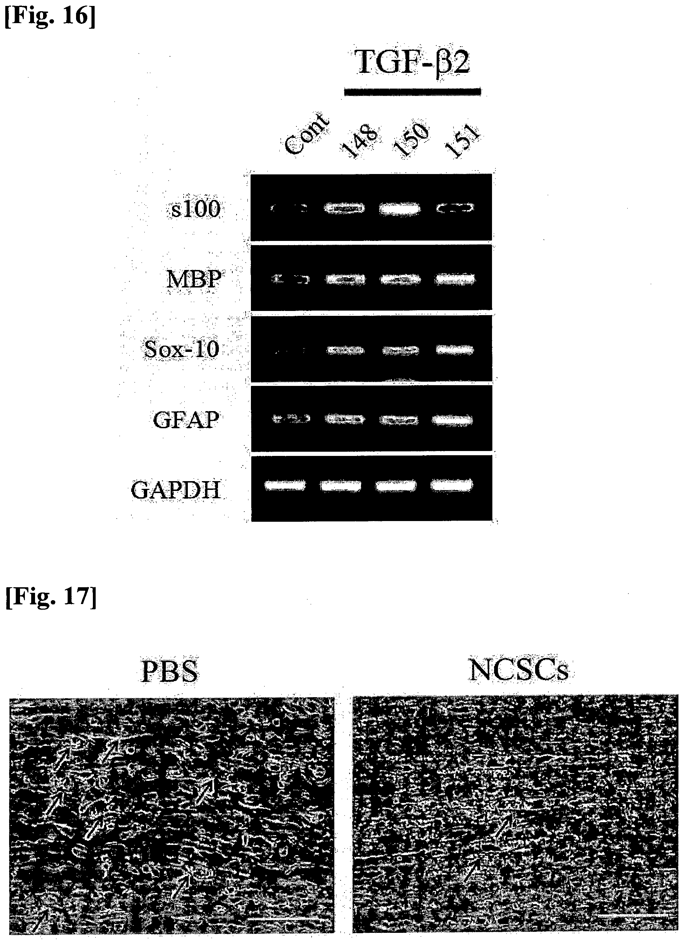

As shown in FIG. 14, after the induced differentiation into Schwann cells, neural crest stem cells showed a multiple structure like an immature peripheral nerve. Neural crest stem cells showed, like Schwann cell, thin cytoplasm with a both-extreme shape. Referring to FIG. 15, neural crest stem cells in immature peripheral nerve continuously expressed p75 and nestin markers. Also, S-100, MBP and GFAP marker, which were not expressed in neural crest stem cells before differentiation, were expressed. That is, neural crest stem cells after differentiation showed the same immunological characteristics as those of Schwann cells. As shown in FIG. 16, compared to before differentiation, after induced differentiation, expression of Schwann cell specific mRNA, such as S-100, MBP, and Sox10 in neural crest stem cells was significantly increased.

### Example 7 Nerve regeneration capacity of neural crest stem cells cultured by amplification in vitro

To confirm peripheral nerve regeneration capacity of neural crest stem cells in vivo, sciatic nerve of 9-week old male BALB/c nu/nu nude mouse was mashed by using a forceps to induce an injury. Three days after the sciatic nerve injury, 2-million neural crest stem cells were injected into sciatic nerve via a syringe. A mouse injected with PBS instead of neural crest stem cell was used as a control. Four weeks after the injection of neural crest stem cells, the mouse was scarified, and then sciatic nerve thereof was collected and fixed with 2% formalin solution. Peripheral nerve regeneration capacity and differentiation into Schwann cell in vivo of neural crest stem cells injected into sciatic nerve were analyzed by hematoxylin eosin staining and immunofluorescence staining. Immunofluorescence staining was performed as follows: a paraffin fragment was blocked using isotype matched serum at room temperature for 40 minutes, and then, reacted with human-specific mitochondria antigen (hMT, Abcam) antibodies at a temperature of 37°C for 2 hours. The result was washed three times with PBS, and then, reacted with isotype matched Dylight 488-conjugated secondary antibodies at room temperature for 40 minutes. Thereafter, the cells were reacted with S-100 or MBP, and then, reacted with isotype matched Dylight 594-conjugated secondary antibodies at room temperature for 40 minutes. Thereafter, nuclei were control-stained using 10 mg/ml DAPI, and then, ProLongGold antifade reagent was mounted thereon, and an image thereof was obtained by using a confocal scan microscope. Functional recovery of peripheral nerve due to injected neural crest stem cells was evaluated by a nerve conduction test.

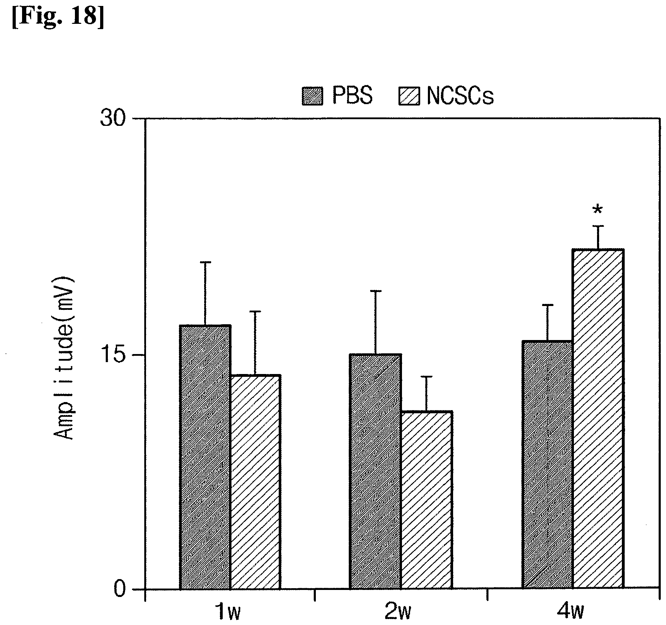

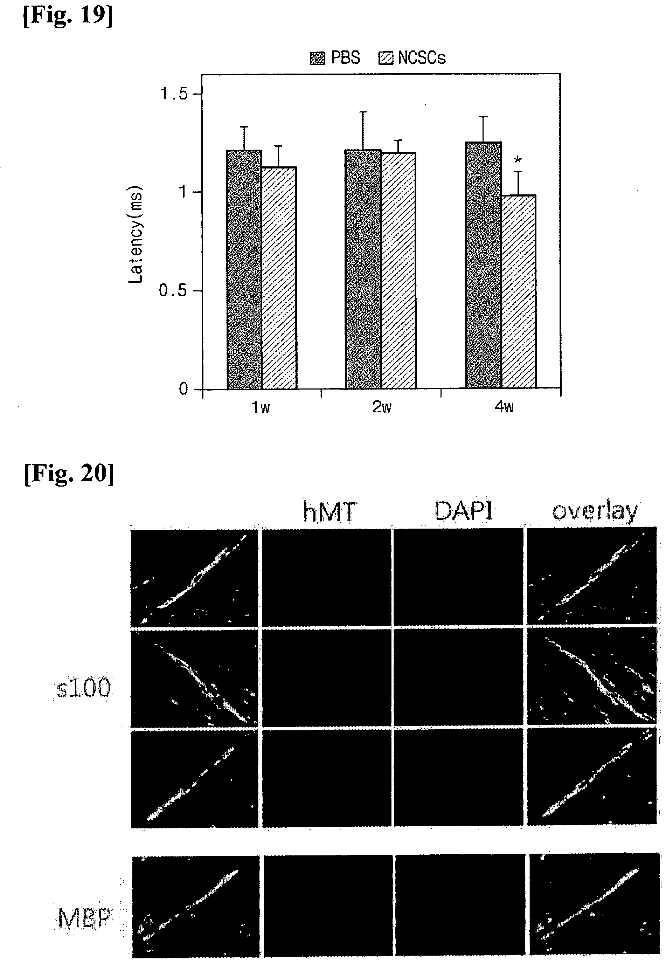

As shown in FIG. 17, when only PBS was injected into sciatic nerve, fibrosis proceeded among nerve fibers, and myelin digestion chamber (indicated as an arrow), which is formed by destructing nerve fiber, was frequently observed. However, when neural crest stem cell (NCSCs) was injected into sciatic nerve, fibrosis and myelin digestion chamber among nerve fibers were significantly decreased. As shown in FIGS. 18 and 19, compared to the control (PBS), neural crest stem cell (NCSCs)-injected sciatic nerve showed a significant increase in nerve conduction amplitude 4 days after the injury (p<0.05)(FIG. 18). Also, after nerve stimulus, the neural crest stem cell (NCSCs)-injected sciatic nerve showed a significant decrease in latency (p<0.05) (FIG. 19). As shown in FIG. 20, it was confirmed that the injected human neural crest stem cells overlayed long nerve fibers, that the cells were injected human cells that are positive to hMT, and that hMT positive human cells expressed S-100 and MBP which are Schwann cell-specific markers, indicating that neural crest stem cells were differentiated into Schwann cell.

## Claims

1. A method of culturing peripheral nerve-derived neural crest stem cells, the method comprising:
culturing peripheral nerve fragments after embedding the peripheral nerve fragments into a hydrogel; and
breaking down only the hydrogel to collect neural crest stem cells that have migrated into the hydrogel from the peripheral nerve fragment and proliferated in the hydrogel.

2. The method of claim 1, wherein the peripheral nerve is the peripheral nerve obtained from an adult body.

3. The method of claim 1, wherein the peripheral nerve-derived neural crest stem cells have at least one immunological profile selected from the group consisting of
(i) an immunological characteristic that is positive to at least one of neural crest stem cell markers selected from the group consisting of nestin, p75, sox-10, and CD56;
(ii) an immunological characteristic that is positive to at least one of mesenchymal stem cell markers selected from the group consisting of CD29, CD44, CD73, CD90, and CD105;
(iii) an immunological characteristic that is positive to at least one of pericyte markers selected from the group consisting of CD140b, CD146, and SMA (α-smooth muscle actin);
(iv) an immunological characteristic that is negative to at least one of hematopoietic cell markers selected from the group consisting of CD34 and CD45;
(v) an immunological characteristic that is negative to at least one of vascular endothelial cell markers selected from the group consisting of CD31 and CD34; and
(vi) an immunological characteristic that is negative to at least one of nervous system cell markers selected from the group consisting of NF (neurofilament), S-100, GFAP (glial fibrillary acid protein), and MBP (myelin basic protein).

4. The method of claim 1, wherein the hydrogel is a collagen hydrogel, and the hydrogel comprises 0.5 to 5.0 mg/ml of collagen.

5. The method of claim 4, wherein the collagen hydrogel is broken down by treatment with a collagenase.

6. The method of claim 1, wherein the method comprises
1) a first step of embedding the peripheral nerve fragment into a collagen hydrogel;
2) a second step of adding a growth culture medium thereto to perform a three-dimensional organ culture in a shaker having a speed of 5 to 30 rpm ;
3) a third step of treating with collagenase to break down only the collagen hydrogel to collect the peripheral nerve fragments and neural crest stem cells which have migrated into the hydrogel from the peripheral nerve fragment and proliferated in the collagen hydrogel; and
4) a fourth step of amplifying the collected neural crest stem cells by monolayer culture.

7. The method of claim 6, wherein the peripheral nerve fragments collected in the third step are embedded into the hydrogel of the first step and cultured.

## Patentansprüche

1. Verfahren zum Kultivieren von Neuralleisten-Stammzellen, die von peripheren Nerven abgeleitet sind, wobei das Verfahren umfasst:
Kultivieren von peripheren Nervenfragmenten nach dem Einbetten der peripheren Nervenfragmente in ein Hydrogel; und
Zersetzen nur des Hydrogels zur Gewinnung der Neuralleisten-Stammzellen, die vom peripheren Nervenfragment in das Hydrogel migriert sind und sich im Hydrogel vermehrt haben.

2. Verfahren nach Anspruch 1, wobei der periphere Nerv ein von einem erwachsenen Körper gewonnener peripherer Nerv ist.

3. Verfahren nach Anspruch 1, wobei die von peripheren Nerven abgeleiteten Neuralleisten-Stammzellen wenigstens ein immunologisches Profil aufweisen, das aus der Gruppe ausgewählt wird, die Folgendes umfasst:
(i) ein immunologisches Merkmal, das positiv gegenüber wenigstens einem der Neuralleisten-Stammzellenmarker ist, die aus der Gruppe ausgewählt werden, die aus Nestin, p75, Sox-10 und CD56 besteht;
(ii) ein immunologisches Merkmal, das positiv gegenüber wenigstens einem der Marker von mesenchymalen Stammzellen ist, die aus der Gruppe ausgewählt werden, die aus CD29, CD44, CD73, CD90 und CD105 besteht;
(iii) ein immunologisches Merkmal, das positiv gegenüber wenigstens einem der Perizytenmarker ist, die aus der Gruppe ausgewählt werden, die aus CD140b, CD146 und SMA (glattmuskulärem α-Actin) besteht;
(iv) ein immunologisches Merkmal, das negativ gegenüber wenigstens einem aus den Markern von hämatopoetischen Zellen ist, die aus der Gruppe ausgewählt werden, die aus CD34 und CD45 besteht;
(v) ein immunologisches Merkmal, das negativ gegenüber wenigstens einem aus den Gefäßendothelzellenmarkern ist, die aus der Gruppe ausgewählt werden, die aus CD31 und CD34 besteht; und
(vi) ein immunologisches Merkmal, das negativ gegenüber wenigstens einem der Nervensystemzellenmarkern ist, die aus der Gruppe ausgewählt werden, die aus NF (Neurofilament), S-100, GFAP (saurem Gliafaserprotein) und MBP (basischem Myelinprotein) besteht.

4. Verfahren nach Anspruch 1, wobei das Hydrogel ein Collagen-Hydrogel ist und das Hydrogel 0,5 bis 5.0 mg/ml Collagen umfasst.

5. Verfahren nach Anspruch 4, wobei das Collagen-Hydrogel durch Behandlung mit einer Collagenase zersetzt wird.

6. Verfahren nach Anspruch 1, wobei das Verfahren umfasst:
1) einen ersten Schritt des Einbettens des peripheren Nervenfragments in ein Collagen-Hydrogel;
2) einen zweiten Schritt des Zusetzens eines Wachstumskulturmediums, um eine dreidimensionale Organkultur in einer Schüttelvorrichtung mit einer Geschwindigkeit von 5 bis 30 Umdrehungen pro Minute durchzuführen;
3) einen dritten Schritt des Behandelns mit Collagenase, um nur das Collagen-Hydrogel zu zersetzen, um die peripheren Nervenfragmente und Neuralleisten-Stammzellen zu gewinnen, die von dem peripheren Nervenfragment in das Hydrogel migriert sind und sich in dem Collagen-Hydrogel vermehrt haben; und
4) einen vierten Schritt des Amplifizierens der gewonnenen Neuralleisten-Stammzellen durch eine Monoschichtkultur.

7. Verfahren nach Anspruch 6, wobei die im dritten Schritt gewonnenen peripheren Nervenfragmente in das Hydrogel des ersten Schritts eingebettet werden und kultiviert werden.

## Revendications

1. Procédé de mise en culture de cellules souches périphériques de la crête neurale dérivées de nerf, le procédé consistant à :
mettre en culture des fragments de nerf périphérique après l'enrobage des fragments de nerf périphérique au sein d'un hydrogel ; et
dégrader uniquement l'hydrogel pour recueillir des cellules souches de la crête neurale qui ont migré à l'intérieur de l'hydrogel à partir du fragment de nerf périphérique et qui ont proliféré dans l'hydrogel.

2. Procédé selon la revendication 1, dans lequel le nerf périphérique est le nerf périphérique obtenu à partir d'un corps d'adulte.

3. Procédé selon la revendication 1, dans lequel les cellules souches périphériques de la crête neurale dérivées de nerf possèdent au moins un profil immunologique choisi parmi le groupe constitué de
(i) une caractéristique immunologique qui est positive envers au moins un des marqueurs de cellules souches de la crête neurale choisi parmi le groupe constitué de la nestine, de p75, de sox-10 et de CD56 ;
(ii) une caractéristique immunologique qui est positive envers au moins un des marqueurs de cellules souches mésenchymateuses choisi parmi le groupe constitué de CD29, de CD44, de CD73, de CD90 et de CD105 ;
(iii) une caractéristique immunologique qui est positive envers au moins un des marqueurs de péricytes choisi parmi le groupe constitué de CD140b, de CD146 et de SMA (α-actine de muscle lisse) ;
(iv) une caractéristique immunologique qui est négative envers au moins un des marqueurs de cellules hématopoïétiques choisi parmi le groupe constitué de CD34 et de CD35 ;
(v) une caractéristique immunologique qui est négative envers au moins un des marqueurs de cellules endothéliales vasculaires choisi parmi le groupe constitué de CD31 et de CD34 ; et
(vi) une caractéristique immunologique qui est négative envers au moins un des marqueurs de cellules du système nerveux choisi parmi le groupe constitué de NF (neurofilament), de S-100, de GFAP (protéine acide fibrillaire gliale) et de MBP (protéine basique de la myéline).

4. Procédé selon la revendication 1, dans lequel l'hydrogel est un hydrogel à base de collagène, et l'hydrogel comprend entre 0,5 et 5,0 mg/ml de collagène.

5. Procédé selon la revendication 4, dans lequel l'hydrogel à base de collagène est dégradé par traitement avec une collagénase.

6. Procédé selon la revendication 1, dans lequel le procédé comprend
1) une première étape d'enrobage du fragment de nerf périphérique à l'intérieur d'un hydrogel à base de collagène ;
2) une seconde état d'addition d'un milieu de culture de croissance à celui-ci pour effectuer une culture d'organe à trois dimensions dans un agitateur-secoueur possédant une vitesse comprise entre 5 et 30 rpm ;
3) une troisième étape de traitement avec la collagénase pour dégrader uniquement l'hydrogel à base de collagène afin de recueillir les fragments de nerf périphérique et les cellules souches de la crête neurale qui ont migré à l'intérieur de l'hydrogel à partir du fragment de nerf périphérique et qui ont proliféré dans l'hydrogel à base de collagène ; et
4) une quatrième étape d'amplification des cellules souches de la crête neurale recueillies par culture monocouche.

7. Procédé selon la revendication 6, dans lequel les fragments de nerf périphérique recueillis dans la troisième étape sont incorporés dans l'hydrogel de la première étape et sont cultivés.
